# EUROPEAN PATENT APPLICATION

(11) **EP 4 368 289 A1**
(43) Date of publication of application: **15.05.2024**
(21) Application number: 22837856.8
(22) Date of filing: 21.06.2022
(51) Int. Cl.: B01L 3/00, B01L 7/00, C12Q 1/686

(54) **PCR MODULE**

(30) Priority: 08.07.2021 KR 20210090030
(71) Applicant: Optolane Technologies Inc., Bundang-gu Seongnam-si, Gyeonggi-do 13494 (KR)
(72) Inventor: CHO, Seung Sun, Ansan-si Gyeonggi-do 15524 (KR); CHOI, An Shik, Seoul 05501 (KR); KWAK, Byoung Joo, Yongin-si Gyeonggi-do 16909 (KR); OLEKSANDROV, Sergiy, Suwon-si Gyeonggi-do 16427 (KR)
(74) Representative: Schiweck Weinzierl Koch Patentanwälte Partnerschaft mbB
(86) International application number: PCT/KR2022/008788
(87) International publication number: WO 2023/282502

(57) **Abstract**

Disclosed is a PCR module in which a sample can be injected using the capillary effect. The PCR module comprises: a microfluidic chamber which includes an inlet part for inputting a sample and is manufactured by injection molding; a well array which is disposed on the lower surface of the microfluidic chamber and includes a plurality of micro-wells of which the upper and lower portions are perforated; and a capillary member providing a path so that the sample input through the inlet part reaches the micro-wells through the capillary effect. When moving a PCR solution to a reaction space using the capillary effect and filling the reaction space with the solution, it is possible to prevent air pockets from forming in the corners or edge regions of the well array which is the reaction space. Accordingly, it is possible to prevent errors caused by air pockets from occurring in PCR test results. In addition, since the well array is located on a CMOS photosensor array, PCR reactions can be measured in real time.

## Description

### Technical Field

The present application claims the benefit of priority based on Korean Patent Application No. 10-2021-0090030 filed on July 8, 2021, the entire disclosure of which is incorporated as a part of this specification.

The present disclosure relates to a PCR module, and more particularly, to a PCR module that prevents occurrence of air pockets in corners or edge areas of a reaction space of a microfluidic chamber while simultaneously enabling real-time PCR measurement.

### Background Art

Gene amplification technology is an essential process in molecular diagnosis, and is a technology that repeatedly copies and amplifies a specific base sequence of trace amounts of deoxyribonucleic acid (DNA) or ribonucleic acid (RNA) in a sample. Among them, polymerase chain reaction (PCR) is a representative gene amplification technology and consists of three steps, which are DNA denaturation step, primer annealing step, and DNA replication step, and since each step depends on the temperature of the sample, DNA may be amplified by repeatedly changing the temperature of the sample.

Previously, PCR was typically performed in 96 or 384-well microplates. When higher throughput is required, PCR methods in conventional microplates are not cost effective or efficient. Meanwhile, reducing the PCR reaction volume may reduce the consumption of reagents and reduce the amplification time at the reduced thermal mass of the reaction volume. This strategy may also be implemented in an array format (m x n), resulting in implementing many smaller reaction volumes. In addition, use of either array allows for scalable high-throughput analysis with increased quantitative sensitivity, dynamic range and specificity.

To date, digital polymerase chain reaction (dPCR) has been performed using array formats. Results from dPCR maybe used to detect and quantify the concentration of rare alleles, provide absolute quantification of nucleic acid samples, and also measure fold changes at low nucleic acid concentrations. In general, increasing the number of replicates increases the accuracy and reproducibility of dPCR results.

The array format of most quantitative polymerase chain reaction (qPCR) platforms is designed for sample-by-sample analysis, where PCR results must be addressable for subsequent analysis. However, for dPCR, the specific location or well of each PCR result is not critical, and only the number of positive and negative copies per sample may be analyzed.

In dPCR, a solution containing a relatively small number of target polynucleotides or nucleic acid sequences may be divided into small test samples, whereby each sample generally contains either one molecule of the target nucleotide sequence or no molecules of the target nucleotide sequence. When the samples are then thermally cycled in a PCR protocol, procedure, or experiment, samples containing the target nucleotide sequence are amplified and produce a positive detection signal, while samples that do not contain any target nucleotide sequence are not amplified and produce no detection signal.

In the case of such digital PCR, the size of the reaction space is very small and the number thereof is very large, so it is difficult to introduce a sample into each reaction space.

In addition, since the sample is not injected to the corner or edge area of the reaction space, air pockets may form in the corner or edge area of the reaction space. Air pockets expand or contract due to temperature changes during the PCR process, causing errors in test results.

The existing main dPCR method uses end-point PCR, which separates the process of introducing the sample into the reaction space and the process of amplifying the base sequence, so it is not possible to measure the reaction in which the base sequence is amplified in real time in each reaction space.

### Disclosure of the Invention

### Technical Goals

Accordingly, a technical challenge of the present disclosure is to solve these conventional problems, and an object of the present disclosure is to provide a PCR module that allows real-time PCR measurement while preventing the occurrence of air pockets at corners or edge areas of a reaction space of a microfluidic chamber by injecting a sample into the reaction space using the capillary principle.

### Technical Solutions

In order to realize the object of the present disclosure described above, a PCR module according to an embodiment of the present disclosure includes a microfluidic chamber including an inlet unit formed for introducing a sample, and capable of being manufactured by injection molding; a well array including a plurality of micro-wells with upper and lower parts penetrated, and disposed on a lower surface of the microfluidic chamber; and a capillary member configured to provide a path so that the sample introduced through the inlet unit reaches the micro-well by capillary action.

### Advantageous Effects

According to this PCR module, when a PCR solution is moved to the reaction space of the microfluidic chamber using the capillary principle and the solution fills the reaction space, it is possible to prevent air pockets from being formed in the corners or edge areas of the well array disposed in the reaction space. Accordingly, it is possible to prevent errors caused by air pockets in PCR test results. Additionally, since the well array is located above the CMOS photosensor array, it is possible to measure real-time PCR reactions.

### Brief Description of Drawings

FIG. 1 is a perspective view for explaining a PCR module according to an embodiment of the present disclosure.
FIG. 2 is an exploded rear perspective view for explaining the PCR module shown in FIG. 1.
FIG. 3 is an exploded perspective view for explaining a capillary member shown in FIG. 1.
FIG. 4 is a cross-sectional view for explaining the PCR module shown in FIG. 1.

### Best Mode for Carrying Out the Invention

In order to realize the object of the present disclosure described above, a PCR module according to an embodiment of the present disclosure includes a microfluidic chamber including an inlet unit formed for introducing a sample, and capable of being manufactured by injection molding; a well array including a plurality of micro-wells with upper and lower parts penetrated, and disposed on a lower surface of the microfluidic chamber; and a capillary member configured to provide a path so that the sample introduced through the inlet unit reaches the micro-well by the capillary action.

In an embodiment, the capillary member may include a first tape disposed between the microfluidic chamber and the well array, and including an inlet hole formed corresponding to the inlet unit, an input hole formed corresponding to the well array, and a connection hole having a width narrower than a diameter of the inlet hole and configured to connect the inlet hole and the input hole.

In an embodiment, the first tape may have a shape of a rectangular shape and a circular shape superimposed on a corner area of the rectangular shape, wherein the rectangular shape may correspond to the well array and the circular shape may correspond to the inlet unit of the microfluidic chamber.

In an embodiment, the input hole may be formed in the rectangular shape, the inlet hole may be formed in the circular shape, and the connection hole may be formed in an area where the rectangular shape and the circular shape are superimposed.

In an embodiment, the first tape may include a double-sided tape with adhesive layers formed on a surface in contact with the microfluidic chamber and a surface in contact with the well array, respectively.

In an embodiment, the input hole may have a rectangular shape larger than the rectangular shape of the well array to expose the micro-wells of the well array.

In an embodiment, the well array may have four edge areas attached to the first tape.

In an embodiment, a side surface forming the connection hole of the first tape may be hydrophilic treated.

In an embodiment, the capillary member may further include a second tape disposed on a lower side of the well array and covering the connection hole and the input hole formed in the first tape, wherein the second tape may correspond to the circular shape of the first tape.

In an embodiment, a surface of the second tape corresponding to the connection hole may be hydrophilic treated.

In an embodiment, a thickness of the second tape may be the same as a thickness of the well array.

In an embodiment, the second tape may be a single-sided tape with an adhesive layer formed on a surface in contact with the first tape.

In an embodiment, the microfluidic chamber may include polydimethylsiloxane (PDMS) material.

In an embodiment, the microfluidic chamber may include; a base member of a rectangular shape including a first flat unit of a rectangular shape, a support hole of a rectangular shape formed in a central area of the first flat unit, and a hole of a circular shape formed in a corner area of the first flat unit; and a tower member of a rectangular shape including a second flat unit of a rectangular shape, a membrane switch of a dish shape, and an inlet unit of a closed-loop shape, and disposed on the base member.

In an embodiment, the PCR module may further include a CMOS photosensor array disposed below the well array and photographing reaction images of the sample filled in the micro-wells of the well array in real time.

### Modes for Carrying Out the Invention

Hereinafter, the present disclosure will be described in more detail with reference to the attached drawings. Since the present disclosure may be variously modified and may have various forms, specific embodiments will be illustrated in the drawings and described in detail in the detailed description. However, this is not intended to limit the present disclosure to a specific disclosed form, and it should be understood that all modifications, equivalents, and substitutes are included in the spirit and scope of the present disclosure.

In describing each drawing, similar reference numerals are used for similar components. In the attached drawings, the dimensions of structures are enlarged from the actual sizes for clarity of the present disclosure.

Terms such as first and second may be used to describe various components, but the components should not be limited by the terms. The terms are used only for the purpose of distinguishing one component from another. For example, a first component may be referred to as a second component without departing from the scope of the present disclosure, and similarly, the second component may also be referred to as a first component. Singular expressions include plural expressions unless the context clearly means otherwise.

In this specification, it should be understood that terms such as "include" or "have" are intended to designate the presence of features, numbers, steps, operations, components, parts, or combinations thereof described in the specification, but do not preclude the possibility of the presence or addition of one or more other features, numbers, steps, operations, components, parts, or combinations thereof.

In addition, unless otherwise defined, all terms used herein, including technical or scientific terms, have the same meaning as those generally understood by a person skilled in the art to which the present disclosure pertains. Terms defined in dictionaries generally used should be construed to have meanings matching contextual meanings in the related art and are not to be construed as an ideal or excessively formal meaning unless otherwise defined herein.

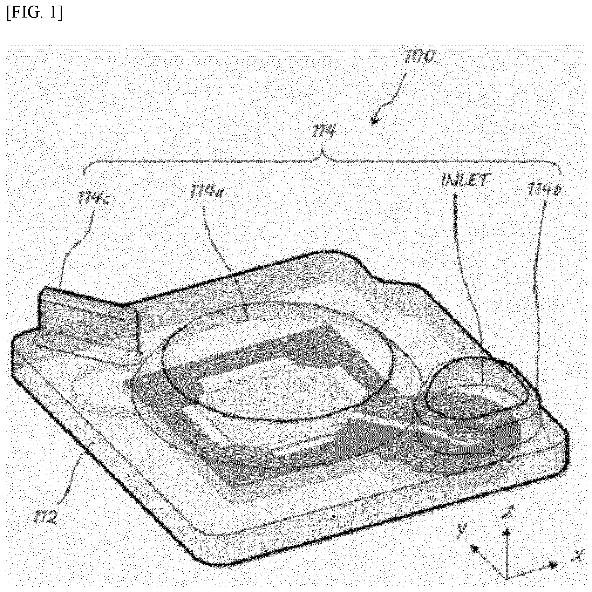

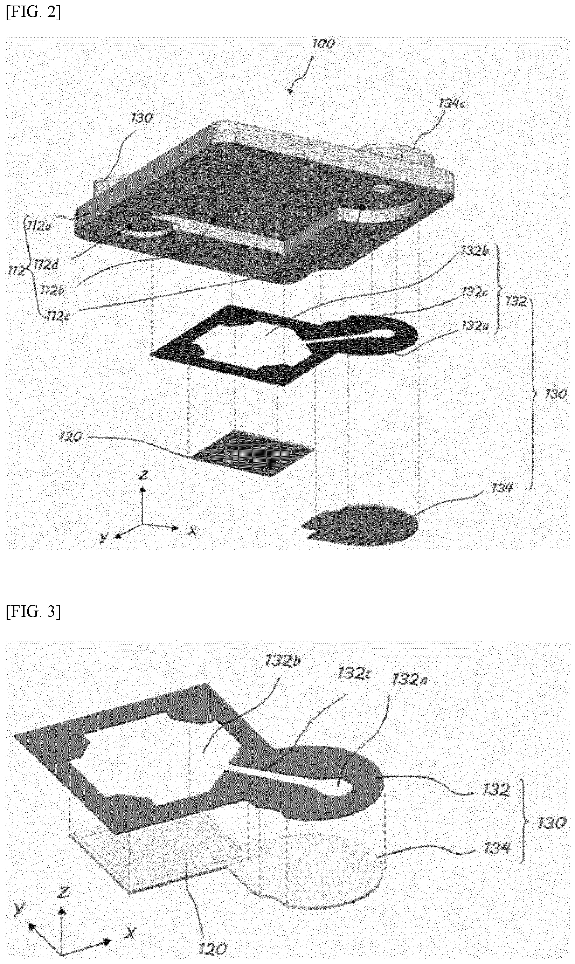

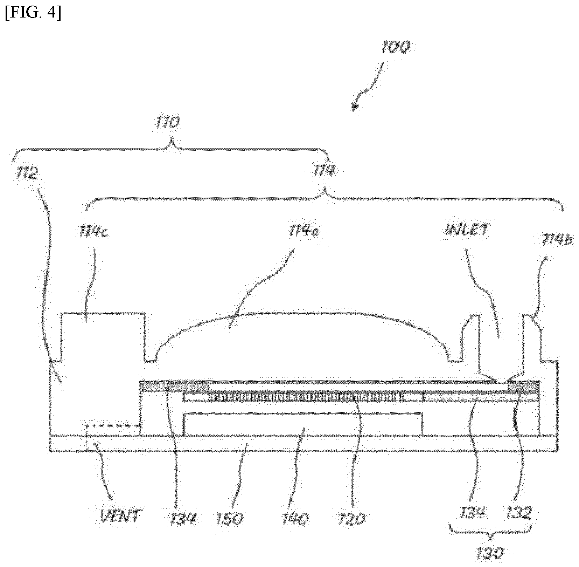

FIG. 1 is a perspective view for explaining a PCR module 100 according to an embodiment of the present disclosure. FIG. 2 is an exploded rear perspective view for explaining the PCR module 100 shown in FIG. 1. FIG. 3 is an exploded perspective view for explaining a capillary member 130 shown in FIG. 1. FIG. 4 is a cross-sectional view for explaining the PCR module 100 shown in FIG. 1.

Referring to FIGS. 1 to 4, the PCR module 100 according to an embodiment of the present disclosure includes a microfluidic chamber 110, a well array 120, and a capillary member 130.

The microfluidic chamber 110 includes a base member 112 and a tower member 114. The base member 112 and the tower member 114 may be formed integrally. In a bottom area of the microfluidic chamber 110, a space of a receding shape is formed to accommodate the well array 120 and the capillary member 130. The microfluidic chamber 110 may include a transparent and elastic material. For example, the microfluidic chamber 110 may include polydimethylsiloxane (PDMS) material. Accordingly, the microfluidic chamber 110 may be manufactured by injection molding.

The base member 112 includes a first flat unit 112a of a rectangular shape, a support hole 112b of a rectangular shape formed in a central area of a lower part of the first flat unit 112a, a first flat hole 112c of a circular shape formed in a first corner area of the lower part of the first flat unit 112a, and a second flat hole 112d of a circular shape formed in a second corner area of the lower part of the first flat unit 112a. The first corner area and the second corner area face each other. The support hole 112b may correspond to the well array 120, the first flat hole 112c may correspond to an inlet unit through which a sample is injected, and the second flat hole 112d may correspond to an outlet unit through which the sample is discharged.

The tower member 114 includes a membrane switch 114a of a dish shape and an inlet unit 114b of a closed-loop shape, and is disposed on the base member 112.

The membrane switch 114a is disposed in a central area of the tower member 114 and protrudes upward. A lower area of the membrane switch 114a corresponds to the support hole of the base member 112. Accordingly, in the bottom part of the microfluidic chamber 110, a space of a receding shape is formed. A CMOS image sensor and a well array 120 may be accommodated in the receding space.

The inlet unit 114b has a fence shape and is formed for inputting a sample. The inlet unit 114b protrudes upward on one side of the membrane switch 114a and blocks the sample from flowing out to the outside. An INLET is formed in a central area of the inlet unit 114b in the vertical direction of the base member 112. The sample is injected into the well array 120 via the capillary member 130 through the INLET.

The tower member 114 may further include a grip unit 114c protruding upward from the observer's viewpoint on the other side of the membrane switch 114a. The grip unit 114c may be disposed to face the inlet unit 114b with respect to the membrane switch 114a. The height of the grip unit 114c may be higher than the height of the inlet unit 114b. The height of the inlet unit 114b and the height of the membrane switch 114a may be the same.

The well array 120 includes a plurality of micro-wells with upper and lower parts penetrated and may be disposed on a lower surface of the microfluidic chamber 110. The well array 120 may be disposed above a CMOS photosensor array 140 and inserted into a substrate hole formed in a PCB 150. The well array 120 may include a plurality of micro wells. The shape, dimensions, and number of microwells may vary. Each of the microwells may accommodate an analysis sample, such as a powder sample or a liquid sample. The analysis sample is a specific component for analyzing biological substances. In other words, the analysis sample is a component for quantitative or qualitative analysis of a specific biological material, such as protein, DNA, RNA, etc, which refers to primers, probes, antibodies, aptamers, DNA or RNA polymerase, etc., and in particular, refers to a component necessary to perform real-time polymerase chain reaction, constant temperature enzyme reaction, ligase chain reaction (LCR), or the like.

The capillary member 130 includes a first tape 132 disposed between the microfluidic chamber 110 and the well array 120 and a second tape 134 disposed on a lower side of the well array 120, and provides a path so that the sample introduced through the inlet unit 114b reaches the micro-well by the capillary action. Here, the capillary action refers to the property of moving along a narrow space such as a thin tube or porous material regardless of external forces such as gravity.

The first tape 132 may have a shape of a rectangular shape and a circular shape superimposed on a corner area of the rectangular shape, wherein the rectangular shape may correspond to the well array 120, and the circular shape may correspond to the inlet unit 114b of the microfluidic chamber 110.

The first tape 132 may include an inlet hole 132a formed corresponding to the inlet unit 114b, an input hole 132b formed corresponding to the well array 120, and a connection hole 132c having a width narrower than a diameter of the inlet hole 132a and configured to connect the inlet hole 132a and the input hole 132b. The input hole 132b may be formed in the rectangular shape, the inlet hole 132a may be formed in the circular shape, and the connection hole 132c may be formed in an area where the rectangular shape and the circular shape are superimposed. The input hole 132b may have a rectangular shape larger than the rectangular shape of the well array 120 and expose the micro-wells of the well array 120.

The first tape 132 may include a double-sided tape with adhesive layers formed on a surface in contact with the microfluidic chamber 110 and a surface in contact with the well array 120, respectively. Accordingly, the well array 120 may have four edge areas attached to the first tape 132. A side surface forming the connection hole 132c of the first tape 132 may be hydrophilic treated. Accordingly, the sample injected through the INLET may reach the well array 120 more smoothly.

The second tape 134 may be disposed on the lower side of the well array 120 and may cover the connection hole 132c and the input hole 132b formed in the first tape 132. The second tape 134 may correspond to the circular shape of the first tape 132. A surface of the second tape 134 corresponding to the connection hole 132c may be hydrophilic treated. Accordingly, the sample injected through the INLET may reach the well array 120 more smoothly. The second tape 134 may be a single-sided tape with an adhesive layer formed on a surface in contact with the first tape 132.

According to the present disclosure, by forming the capillary member 130 with the first tape 132 disposed between the microfluidic chamber 110 and the well array 120 and the second tape 134 disposed on the lower side of the well array 120, it is possible to prevent air pockets from being created in the corners or edge areas of the well array 120.

Typically, air pockets expand or contract due to temperature changes during the PCR process, causing errors in PCR test results. However, according to the present disclosure, when the sample is moved by the capillary action using the capillary member 130 and the sample fills the reaction space of the microfluidic chamber 110, the creation of air pockets is blocked at the corners or edge areas of the well array 140, which is the reaction space. Therefore, it is possible to prevent errors in the test results due to air pockets in the PCR test results. In addition, since the well array 120 is located above the CMOS photosensor array 140, real-time PCR reactions may be measured.

The PCR module 100 according to the present disclosure may further include the CMOS photosensor array 140. The CMOS photosensor array 140 may be disposed below the well array 120 and may photograph images of a product of the PCR reaction performed by filling the micro-wells of the well array 120 in real time.

The CMOS photosensor array 140 may be disposed by being inserted into a substrate hole formed in the PCB 150. The CMOS photosensor array 140 may receive the emitted light and photograph the product of the PCR reaction performed in the PCR device in real time. In other words, the CMOS photosensor array 140 detects fluorescence (emission light) generated from a plurality of probes by excitation light. Detection of the above-mentioned fluorescence may be performed by a time separation method or a wavelength separation method.

In the case of the time separation method described above, as the fluorescent material emits emission light in response to excitation light, the fluorescent sensor array or a single sensor forming the array detects the emission light passing through the emission filter and determines the time constant of the detected emission light to sense the fluorescence.

To this end, the PCR module 100 may further include an emission filter (not shown) that selects light having a predetermined wavelength. The emission filter may be disposed on the CMOS photosensor array 140.

Meanwhile, in the case of the above-described wavelength separation method, as the fluorescent material responds to the excitation light and emits emission light, the fluorescent sensor array or a single sensor forming the array detects the emission light passing through the above-described emission filter and senses the fluorescence through spectral analysis of the detected emission light.

The PCR module 100 according to the present disclosure may further include the PCB 150. The PCB 150 is disposed below the microfluidic chamber 110 and may accommodate the mounted CMOS photosensor array 140. The PCB 150 may be disposed in contact with a bottom edge area of the microfluidic chamber 110.

When a sample is introduced into the INLET, it is supplied to a partial area of the well array 120, an upper area of the partial area, and a lower area of the partial area by the capillary member 130.

In this embodiment, the PCR module 100 may further include a light providing unit (not shown) disposed to irradiate an excitation light toward the probe accommodated in each of the microwells of the well array 120. In an embodiment, the light providing unit may include a light source that emits light, such as a light emitting diode (LED) light source, a laser light source, or the like. Light emitted from the light source passes through or reflects off the micro wells of the well array 120, and in this case, the CMOS photosensor array 140 may detect the optical signal generated by nucleic acid amplification.

As described above, according to the present disclosure, since the PCR module is released with reagents built into the well array, there is no need for separate procedures to set up the reagents, dramatically reducing the possibility of contamination and eliminating the need for separate procedures to prepare for testing.

In addition, in the case of digital real-time PCR, even if the size of the microwells of the well array, which is the reaction space, is very small and the number is very large, it is possible to inject a sample into each of the reaction spaces by the capillary action.

In addition, since the sample is injected to the corner or edge area of the well array, the creation of air pockets in the corner or edge area of the well array, which is the reaction space, is prevented, and the reliability of the test results is improved.

Although described above with reference to examples, those skilled in the art will understand that various modifications and changes may be made to the present disclosure without departing from the spirit and scope of the present disclosure as set forth in the appended claims.

### <Explanation of Symbols>

100 : PCR module 110 : Microfluidic chamber
112a : First flat unit 112b : Support hole
112c : First flat hole 112 : Base member
112d : Second flat hole 114 : Tower member
114a : Membrane switch 114b : Inlet unit
120 : Well array 130 : Capillary member
132 : First tape 132a : Inlet hole
132b : Input hole 132c : Connection hole
134 : Second tape 140 : CMOS photosensor array
150 : PCB

### Industrial Applicability

According to a PCR module of the present disclosure, when a PCR solution is moved to the reaction space of the microfluidic chamber using the capillary principle and the solution fills the reaction space, it is possible to prevent air pockets from being formed in the corners or edge areas of the well array disposed in the reaction space. Accordingly, it is possible to prevent errors caused by air pockets in the PCR test results. In addition, since the well array is located above the CMOS photosensor array, it is possible to measure real-time PCR reactions.

## Claims

1. A PCR module comprising:
a microfluidic chamber comprising an inlet unit formed for introducing a sample, and capable of being manufactured by injection molding;
a well array comprising a plurality of micro-wells with upper and lower parts penetrated, and disposed on a lower surface of the microfluidic chamber; and
a capillary member configured to provide a path so that the sample introduced through the inlet unit reaches the micro-well by capillary action.

2. The PCR module of claim 1, wherein the capillary member comprises a first tape disposed between the microfluidic chamber and the well array, and comprising an inlet hole formed corresponding to the inlet unit, an input hole formed corresponding to the well array, and a connection hole having a width narrower than a diameter of the inlet hole and configured to connect the inlet hole and the input hole.

3. The PCR module of claim 2, wherein the first tape has a shape of a rectangular shape and a circular shape superimposed on a corner area of the rectangular shape, and
wherein the rectangular shape corresponds to the well array, and the circular shape corresponds to the inlet unit of the microfluidic chamber.

4. The PCR module of claim 3, wherein the input hole is formed in the rectangular shape, the inlet hole is formed in the circular shape, and the connection hole is formed in an area where the rectangular shape and the circular shape are superimposed.

5. The PCR module of claim 2, wherein the first tape comprises a double-sided tape with adhesive layers formed on a surface in contact with the microfluidic chamber and a surface in contact with the well array, respectively.

6. The PCR module of claim 2, wherein the input hole has a rectangular shape larger than the rectangular shape of the well array to expose the micro-wells of the well array.

7. The PCR module of claim 2, wherein the well array has four edge areas attached to the first tape.

8. The PCR module of claim 2, wherein a side surface forming the connection hole of the first tape is hydrophilic treated.

9. The PCR module of claim 2, wherein the capillary member further comprises a second tape disposed on a lower side of the well array and covering the connection hole and the input hole formed in the first tape, and
wherein the second tape corresponds to the circular shape of the first tape.

10. The PCR module of claim 9, wherein a surface of the second tape corresponding to the connection hole is hydrophilic treated.

11. The PCR module of claim 9, wherein a thickness of the second tape is the same as a thickness of the well array.

12. The PCR module of claim 9, wherein the second tape is a single-sided tape with an adhesive layer formed on a surface in contact with the first tape.

13. The PCR module of claim 1, wherein the microfluidic chamber comprises polydimethylsiloxane (PDMS) material.

14. The PCR module of claim 1, wherein the microfluidic chamber comprises:
a base member of a rectangular shape comprising a first flat unit of a rectangular shape, a support hole of a rectangular shape formed in a central area of the first flat unit, and a hole of a circular shape formed in a corner area of the first flat unit; and
a tower member of a rectangular shape comprising a second flat unit of a rectangular shape, a membrane switch of a dish shape, and an inlet unit of a closed-loop shape, and disposed on the base member.

15. The PCR module of claim 1, further comprising:
a CMOS photosensor array disposed below the well array and photographing reaction images of the sample filled in the micro-wells of the well array in real time.
